(19) Europäisches Patentamt
European Patent Office
Office européen des brevets

(11) Veröffentlichungsnummer: **0 094 565**
**B1**

(12) # EUROPÄISCHE PATENTSCHRIFT

(45) Veröffentlichungstag der Patentschrift: **16.10.85**

(51) Int. Cl.⁴: **C 07 D 265/30**

(21) Anmeldenummer: **83104414.4**

(22) Anmeldetag: **05.05.83**

(54) **Verfahren zur Herstellung von cis-2,6-Dimethylmorpholin.**

(30) Priorität: **13.05.82 DE 3217964**

(43) Veröffentlichungstag der Anmeldung: **23.11.83 Patentblatt 83/47**

(45) Bekanntmachung des Hinweises auf die Patenterteilung: **16.10.85 Patentblatt 85/42**

(84) Benannte Vertragsstaaten: **AT BE CH DE FR GB IT LI NL SE**

(56) Entgegenhaltungen:
**FR - A - 2 325 647**
**GB - A - 883 220**
**US - A - 3 083 202**

(73) Patentinhaber: **BASF Aktiengesellschaft, Carl-Bosch-Strasse 38, D-6700 Ludwigshafen (DE)**

(72) Erfinder: **Goetz, Norbert, Dr., Schoefferstrasse 25, D-6520 Worms 1 (DE)**
Erfinder: **Girgensohn, Bjoern, Dr., Neckarpromenade 38, D-6800 Mannheim 1 (DE)**
Erfinder: **Zanker, Fritz, Dr., Denkstrasse 27, D-6520 Worms 1 (DE)**

**EP 0 094 565 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Herstellung von Morpholinen durch Wasserabspaltung aus Bis-hydroxyalkyl-aminen in Anwesenheit von sauren Katalysatoren, beispielsweise Schwefelsäure, oder durch Dampfphasen-Dehydratisierung an Aluminiumoxid-Katalysatoren (Houben-Weyl «Methoden der organischen Chemie», 4. Auflage, Georg Thieme-Verlag, Stuttgart, 1966, Band 6/4, S. 510 bis 520) ist seit langem bekannt. In geringerem Masse wird über die Probleme bei der Herstellung sterisch einheitlicher Verbindungen im Zusammenhang mit der Synthese von Morpholinen berichtet, die zwei und mehr Substituenten am Ring tragen und die Bildung von verschiedenen stereoisomeren Verbindungen in unterschiedlichem Masse – in Abhängigkeit von den Reaktionsbedingungen – erwarten lassen. Beispielsweise gibt es mechanistische Untersuchungen zur Cyclisierung von 3,3'-Iminobis-2-butanolen zu 2,3,5,6-Tetramethylmorpholinen in Schwefelsäure (J. Heterocyclic Chemistry *17* (1980), S. 369 bis 372).

Für viele Zwecke ist das cis-Isomere des 2,6-Dimethylmorpholins dem Isomerengemisch vorzuziehen. Beispielsweise beruht die Wirkung von daraus hergestellten Pflanzenschutzmitteln hautpsächlich auf den Verbindungen, die aus dem cis-Isomeren gewonnen wurden.

Eine Methode zur Anreicherung des cis-Gehaltes in einem 2,6-Dimethylmorpholin-Isomerengemisch durch Isomerisierung mit überschüssiger Schwefelsäure oder durch Cyclisierung von Diisopropanolamin mit konzentrierter Schwefelsäure bei 185 bis 220° C ist in der US-PS 3083202 zu finden. Nachteilig ist hierbei die hohe Reaktionstemperatur, da konzentrierte Schwefelsäure bekanntlich (H. Remy, Lehrbuch der anorg. Chemie, 13. Aufl., Akademische Verlagsgesellschaft Geest und Portig K.G., Leipzig, 1970, Band 1, S. 861, 921 und 922) in der Hitze unter Sauerstoffabgabe in schweflige Säure bzw. Schwefeldioxid übergeht und auf organisches Material verkohlend einwirkt. Instabil ist vor allem das freie Amin (Diisopropanolamin und Morpholin), weniger das Salz. Deshalb wird gemäss der genannten US-PS das Amin unter Kühlen und Rühren so langsam zu der vorgelegten Schwefelsäure gegeben, dass die Temperatur der Lösung nicht über 80° C steigt. Erst nachdem alles Amin zur Schwefelsäure gegeben ist, wird auf ca. 200° C erhitzt. Das Verfahren ist energie- und zeitaufwendig (erst Kühlen, währenddem praktisch noch keine Umsetzung im gewünschten Sinne erfolgt, dann Heizen), ausserdem sind die für das Verfahren angegebenen Ausbeuten nach unseren Erfahrungen tatsächlich nicht zu erreichen, weil sich bei Temperaturen ab 190° C auch die Zersetzung des Salzes störend bemerkbar macht. Vielmehr erhält man zahlreiche zum Teil tiefgefärbte Nebenprodukte und die Ausbeuten an 2,6-Dimethylmorpholin liegen deutlich niedriger als beim erfindungsgemässen Verfahren.

Der Erfindung lag daher die Aufgabe zugrunde, ein möglichst wirtschaftliches Verfahren zur Herstellung von 2,6-Dimethylmorpholin in möglichst hoher Ausbeute und mit möglichst hohem cis-Anteil zu entwickeln.

Es wurde nun gefunden, dass man 2,6-Dimethylmorpholin

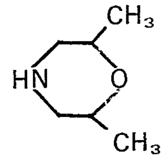

in hoher Ausbeute und mit einem hohen cis-Anteil (75 bis 88%) erhält, wenn man Diisopropanolamin (1,1'-Iminobis)-2-propanol

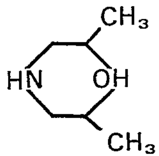

mit einem Wassergehalt von 0 bis 20, vorzugsweise 5 bis 15 Gewichtsprozent, bezogen auf das Amin, gleichzeitig mit überschüssiger (d.h. so viel, dass die Reaktionsmischung sauer ist) konzentrierter Schwefelsäure (90 bis 120, vorzugsweise 98 bis 105%ig), vorzugsweise unter Einhaltung eines Molverhältnisses Amin: Säure wie 1 : (1,0 bis 3,0), so rasch in ein Reaktionsgefäss einführt, dass die Temperatur des Mischung ohne Kühlmassnahmen, d.h. lediglich mit der natürlichen Kühlung durch die umgebende Luft von im allgemeinen Raumtemperatur, in den Bereich von 85 bis 170, vorzugsweise 100 bis 150° C steigt, anschliessend bei einer Temperatur von 150 bis 190, vorzugsweise 170 bis 184° C und einer Verweilzeit von 1 bis 25, vorzugsweise 3 bis 21 h unter Abdestillation des vorhandenen und/oder entstehenden Wassers umsetzt, das gebildete Reaktionsprodukt unter Kühlung in verdünnte Natronlauge (z.B. etwa 10 bis 25%ig) einrührt und dabei zweckmässig einen pH von etwa 12 bis 14 einstellt, die hierbei gebildete organische Phase zur Abtrennung störender Nebenprodukte, vorzugsweise bei Stumpftemperaturen von 50 bis 110° C, im Vakuum destilliert und das erhaltene Destillat (rohes, wasserhaltiges 2,6-Dimethylmorpholin), zweckmässig durch Ausschütteln oder Verrühren mit konzentrierter Natronlauge (ca. 50%ig) bei Temperaturen unter 40° C, trocknet.

Entscheidend für die Ausbeute ist dabei, dass nicht gemäss dem Stand der Technik eine Komponente (Amin oder Schwefelsäure) vorgelegt und die andere zugegeben, sondern dass beide Komponenten gleichzeitig in den Reaktionsraum gegeben werden. Dies geschieht unter Rühren ,doch sind an die Intensität des Rührens keine besonderen Anforderungen zu stellen. Es spielt auch keine Rolle, ob die Komponenten durch die gleiche Öffnung im Reaktionsgefäss zulaufen, so dass sie sich schon mehr oder weniger mischen, bevor sie die heisse Reaktionsmischung im Gefäss erreichen, oder ob sie durch zwei getrennte Öffnungen zugegeben werden.

Beim Arbeiten nach dem erfindungsgemässen Verfahren wird 98 bis 99%iges 2,6-Dimethylmorpholin gewonnen, welches sich problemlos durch fraktionierte Destillation an einer Füllkörperkolonne in reines cis- und reines trans-2,6-Dimethylmorpholin trennen lässt (s. DE-OS 2656747). Bei Gesamtausbeuten an 2,6-Dimethylmorpholin von 90 bis 98% können Anteile an cis-2,6-Dimethylmorpholin von 75 bis 88% erreicht werden, wobei ein Isomerenverhältnis von etwa 88% cis-Verbindung zu 12% trans-Verbindung der Einstellung des thermodynamischen Gleichgewichtes entspricht, wie man anhand von Isomerisierungsversuchen an Hydrierkatalysatoren (s. DE-OS 2938698 und 2830998) bei langen Verweilzeiten im Temperaturbereich von 170 bis 250°C feststellen kann. Höhere cis-Anteile im Reaktionsgemisch (> 88%) erhält man nur auf Kosten der Gesamtausbeute, wenn man gemäss US-PS 3083202 unter recht drastischen Reaktionsbedingungen einen Teil insbesondere des thermodynamisch instabileren trans-2,6-Dimethylmorpholins zerstört. So machen sich beispielsweise unter den in der genannten Schrift angegebenen Reaktionsbedingungen (bevorzugter Temperaturbereich 190 bis 210°C) die Eigenschaften der konzentrierten Schwefelsäure als Oxidationsmittel stark bemerkbar, was an der $SO_2$-Entwicklung während der Reaktion, am Entstehen einer Wassermenge weit über dem Wert der Theorie und an den schlechten Gesamtausbeuten an 2,6-Dimethylmorpholin zu erkennen ist (siehe vorliegendes Vergleichsbeispiel). Die in der oben zitierten US-PS angegebenen Gesamtausbeuten konnten trotz genauestem Einhalten der Reaktionsbedingungen nicht reproduziert werden. Beim erfindungsgemässen Verfahren wird die Umsetzung in einem Temperaturbereich vorgenommen, in dem die Oxidationswirkung der Schwefelsäure noch keine nennenswerte Rolle spielt. Die Vorteile des Verfahrens sind demnach:

1. Hohe Gesamtausbeute an 2,6-Dimethylmorpholin.
2. Hoher Anteil der cis-Verbindung.
3. Geringer Energieaufwand, weil die gesamte Reaktionswärme genutzt wird.
4. Kein Aufwand für eine Kühleinrichtung für das Reaktionsgefäss.
5. Kein aufgewärmtes Kühlwasser.
6. Zeitgewinn, weil die Reaktion bereits während der Zugabe der Komponenten anläuft.
7. Verminderte Korrosion in den Reaktoren aufgrund der tieferen Reaktionstemperatur.
8. Die Trocknung des Rohproduktes mit konzentrierter Natronlauge (30 bis 50, vorzugsweise 45 bis 50%ig) ist besonders ökonomisch, weil die sich dabei verdünnende Natronlauge zum Neutralisieren des Reaktionsgemisches der nächsten Partie Verwendung findet.

Die beiden isomeren 2,6-Dimethylmorpholine lassen sich – wie bereits erwähnt – durch fraktionierte Destillation gut trennen (DE-OS 2656747), wobei sich das unerwünschte trans-2,6-Dimethylmorpholin durch Isomerisierung, vorzugsweise an Hydrierkatalysatoren (DE-OS 2830998 und 2938698), in das Wertprodukt cis-2,6-Dimethylmorpholin überführen lässt. Weniger geeignet ist das in der US-PS 3083202 beschriebene Isomerisierungsverfahren, welches zwangsweise eine unerwünschte Salzfracht als Abfallprodukt liefert.

Das als Ausgangsstoff benutzte Diisopropanolamin ist durch Umsetzung von Ammoniak mit Propylenoxid gut zugänglich (Houben-Weyl, Methoden der organischen Chemie, Band 11/1, S. 311 bis 327).

Die Umsetzung wird kontinuierlich oder diskontinuierlich und – abgesehen von der Schwefelsäure – lösungsmittelfrei durchgeführt.

Das nach dem erfindungsgemässen Verfahren hergestellte cis-2,6-Dimethylmorpholin ist ein wertvolles Zwischenprodukt für Pflanzenschutzmittel (DE-OS 2656747, 2752096 und 2752135).

Die in den folgenden Beispielen genannten Teile bedeuten Gewichtsteile, Raumteile verhalten sich zu ihnen wie Liter zum Kilogramm.

*Beispiel 1:*

Einer Rührapparatur mit einem Volumen von 1000 Raumteilen wurden gleichzeitig unter Rühren

a) eine Mischung aus 266 Teilen Diisopropanolamin und 30 Teilen Wasser und

b) 255 Teile 96%ige Schwefelsäure (Molverhältnis Diisopropanolamin: Schwefelsäure = 1,0:1,25)

zugeführt. Die Zulaufgeschwindigkeit wurde so eingestellt, dass die Temperatur im Reaktionsraum ohne Kühlung zwischen 100 und 120°C blieb. Anschliessend wurde 21 h lang auf 150°C erhitzt, wobei das vorhandene und das sich im Laufe der Reaktion bildende Wasser kontinuierlich abdestilliert wurde. Das Reaktionsprodukt wurde nach dem Abkühlen mit 200 Teilen Wasser verdünnt und zu einer vorgelegten Menge von 800 Teilen 25%iger Natronlauge unter Rühren und Kühlung zugefahren. Der pH-Wert dieser Mischung betrug nach beendeter Zufuhr 14. Es bildeten sich 2 Phasen aus, die organische (obere) Phase wurde abgetrennt und im Vakuum (ca. 100 mbar) destilliert. Man erhielt 521 Teile Destillat (Gemisch aus Wasser und 2,6-Dimethylmorpholin), welches in 2 Stufen – durch Verrühren zunächst mit 300, dann mit 150 Gewichtsteilen 50%iger Natronlauge – entwässert wurde.

Nach dieser Trocknung fielen 228 Teile reines 2,6-Dimethylmorpholin (99%iges Produkt) an, entsprechend einer Ausbeute von 98% der Theorie. Das Isomerenverhältnis betrug 78% cis-/22% trans-Verbindung.

Die bei der Trocknung erhaltene verdünnte Natronlauge wurde beim nächsten Ansatz zur Neutralisation des Reaktionsproduktes eingesetzt.

*Beispiel 2:*

Man arbeitete analog Beispiel 1 bei einem Molverhältnis Diisopropanolamin: Schwefelsäure = 1,0:1,25, erhitzte aber 12 h auf eine Reaktionstemperatur von 170°C. Unter diesen Bedingungen wurde 2,6-Dimethylmorpholin ebenfalls in einer Gesamtausbeute von 98% der Theorie und mit einem Isomerenverhältnis von 78% cis-/22% trans-Verbindung erhalten.

*Beispiel 3:*

Man arbeitete analog Beispiel 1, aber bei einem Molverhältnis Diisopropanolamin: Schwefelsäure = 1,0:1,5 und erhitzte 5 h lang auf 180°C. Unter diesen Reaktionsbedingungen wurde 2,6-Dimethylmorpholin in einer Gesamtausbeute von 96% der Theorie und mit einem Isomerenverhältnis von 80% cis-/20% trans-Verbindung gebildet.

*Beispiel 4:*

Man arbeitete analog Beispiel 1, aber bei einem Molverhältnis Diisopropanolamin: Schwefelsäure = 1,0:2,0 und erhitzte 3 h lang auf 180°C. Unter diesen Reaktionsbedingungen wurde 2,6-Dimethylmorpholin in einer Gesamtausbeute von 94% der Theorie und mit einem Isomerenverhältnis von 84% cis-/16% trans-Verbindung gebildet.

*Beispiel 5:*

Man arbeitete analog Beispiel 1, aber bei einem Molverhältnis Diisopropanolamin: Schwefelsäure = 1,0:3,0 und erhitzte 3 h lang auf 180°C. Unter diesen Reaktionsbedingungen wurde 2,6-Dimethylmorpholin in einer Gesamtausbeute von 91% der Theorie und mit einem Isomerenverhältnis von 88% cis-/12% trans-Verbindung gebildet.

*Beispiel 6:*

a) Erfindungsgemäss

In einen Reaktor mit einem Volumen von 1000 Raumteilen wurden unter Rühren gleichzeitig 245 Teile 96%ige Schwefelsäure und eine Mischung aus 266 Teilen Diisopropanolamin und 30 Teilen Wasser bei intensiver Durchmischung eingefüllt, wobei die Temperatur ohne Kühlen zwischen 100 und 120°C blieb. Danach wurde 5 h lang auf 184°C erhitzt, wobei das vorhandene und das durch die Umsetzung gebildete Wasser (insgesamt 75 Teile; theoretischer Wert: 76 Teile Wasser) kontinuierlich abdestilliert wurde.

Das Reaktionsprodukt liess man nun unter Rühren und Kühlen zu 1000 Teilen 20%iger Natronlauge laufen. Der End-pH-Wert der Mischung betrug 14. Es bildeten sich 2 Phasen aus, die organische (obere) Phase wurde abgetrennt und im Vakuum (ca. 100 mbar) destilliert. Man erhielt 515 Gewichtsteile Destillat (Gemisch aus Wasser und 2,6-Dimethylmorpholin), welches in 2 Stufen – durch Verrühren zunächst mit 300, dann mit 150 Gewichtsteilen 50%iger Natronlauge – getrocknet wurde.

Nach dieser Trocknung fielen 216 Gewichtsteile reines 2,6-Dimethylmorpholin (99%iges Produkt) an, entsprechend einer Ausbeute von 93% der Theorie. Das Isomerenverhältnis betrug 78% cis-/22% trans-Verbindung.

b) Vergleichsversuch

Man arbeitete analog Beispiel 6a, legte aber die Mischung aus Diisopropanolamin und Wasser vor und gab unter Rühren bei Temperaturen von 100 bis 120°C die konzentrierte Schwefelsäure zu. Bei dieser Arbeitsweise wurden unerwünschte Nebenreaktionen (Crack- und Verharzungsprozesse) mit verstärkter Bildung von öligen und teerartigen Nebenprodukten beobachtet, die beim Destillieren des rohen 2,6-Dimethylmorpholins im Destillationsrückstand blieben. Die Gesamtausbeute an 2,6-Dimethylmorpholin betrug hier 78% der Theorie mit einem unveränderten Isomerenverhältnis von 78% cis-/22% trans-Verbindung.

c) Vergleichsversuch

Man arbeitete analog Beispiel 6a, legte aber die konzentrierte Schwefelsäure vor und pumpte bei Temperaturen von 100 bis 120°C die Mischung aus Diisopropanolamin und Wasser zu. Auch bei dieser Verfahrensvariante wurden unerwünschte Nebenreaktionen beobachtet, die sich in einem starken Schäumen beim Abdestillieren des Wassers aus dem Reaktionsraum äusserten. Die Gesamtausbeute an 2,6-Dimethylmorpholin betrug in diesem Fall 81% der Theorie bei unverändertem Isomerenverhältnis von 78% cis-/22% trans-Verbindung.

*Vergleichsbeispiel gem. Beispiel 8 der US-PS 3083202*

In einer Rührapparatur wurden 450 Gewichtsteile konzentrierte Schwefelsäure (96%ig) vorgelegt und unter Rühren und Kühlen 266 Gewichtsteile Diisopropanolamin bei Temperaturen unterhalb von 80°C zugegeben (Molverhältnis Diisopropanolamin: Schwefelsäure = 1:2,2). Anschliessend wurde 3 h lang auf 200°C erhitzt, wobei insgesamt 84 Gewichtsteile Wasser (theoretischer Wert: 54 Gewichtsteile Wasser) abdestilliert wurden. Gleichzeitig war während des gesamten Reaktionsablaufes bei 200°C eine starke Schwefeldioxidentwicklung festzustellen.

Die Aufarbeitung erfolgte analog Beispiel 6a. Man liess das Reaktionsprodukt unter Rühren und Kühlen zu 1840 Gewichtsteilen 20%iger Natronlauge laufen (End-pH-Wert: 14), trennte die organische Phase ab, destillierte diese unter vermindertem Druck und trocknete das Destillat mit 50%iger Natronlauge.

Es wurden 151 Gewichtsteile reines 2,6-Dimethylmorpholin (99%iges Produkt) erhalten, entsprechend einer Ausbeute von 65% der Theorie, das Isomerenverhältnis betrug 88% cis-/12% trans-Verbindung (Soll-Wert für Gesamtausbeute 98%, siehe US-PS 3088202).

Die unter diesen Reaktionsbedingungen gebildete Wassermenge, die weit über dem theoretischen Wert liegt, und die starke $SO_2$-Entwicklung weisen darauf hin, dass die konzentrierte Schwefelsäure in dem angegebenen Temperaturbereich schon in starkem Masse als Oxidationsmittel wirkt.

*Beispiel 7:*

Man arbeitete analog Beispiel 6a, aber mit dem Unterschied, dass die gleichzeitige Zufuhr der Komponenten Schwefelsäure und der Diisopropanolamin/Wasser-Mischung bei 140-145°C vorgenommen wurde. Unter diesen Reaktionsbedingungen wurde 2,6-Dimethylmorpholin ebenfalls in einer Gesamtausbeute von 93% der Theorie

und ein Isomerenverhältnis von 78% cis-/22% trans-Verbindung erhalten.

## Patentansprüche

1. Verfahren zur Herstellung von 2,6-Dimethylmorpholin mit einem hohen cis-Isomerengehalt durch Cyclisieren von Diisopropanolamin mit Schwefelsäure, Aufarbeiten des Reaktionsproduktes mit Natronlauge, Destillieren und Trocknen des erhaltenen Rohproduktes, dadurch gekennzeichnet, dass Diisopropanolamin mit einem Wassergehalt von 0 bis 20 Prozent und überschüssige 90 bis 120 prozentige Schwefelsäure gleichzeitig unter Rühren ohne Kühlmassnahmen so in den Reaktionsraum dosiert werden, dass die Temperatur im Reaktionsmedium auf 85 bis 170°C steigt, und dass anschliessend 1 bis 25 h unter Abdestillieren von Wasser auf 150 bis 190°C erhitzt wird.

2. Verfahren gemäss Anspruch 1, dadurch gekennzeichnet, dass ein Molverhältnis Diisopropanolamin: Schwefelsäure von 1:1,0 bis 1:3,0 eingehalten wird.

3. Verfahren gemäss Anspruch 1, dadurch gekennzeichnet, dass das wasserhaltige 2,6-Dimethylmorpholin mit konzentrierter Natronlauge unter 40°C getrocknet wird.

4. Verfahren gemäss Anspruch 3, dadurch gekennzeichnet, dass die durch Trocknung des 2,6-Dimethylmorpholins mit konzentrierter Natronlauge erhaltene verdünnte Natronlauge bei folgenden Reaktionsansätzen zur Aufarbeitung des schwefelsauren Umsetzungsproduktes verwendet wird.

## Claims

1. A process for the preparation of 2,6-dimethylmorpholine containing a high proportion of the cis-isomer by cyclization of diisopropanolamine with sulfuric acid, working up the reaction mixture with sodium hydroxide solution, and distilling and drying the crude product obtained, wherein diisopropanolamine containing from 0 to 20 percent of water, and excess 90 to 120 percent strength sulfuric acid are simultaneously metered in such a manner into the reaction space, while stirring but without cooling, that the temperature of the reaction medium increases to 85 to 170°C, and the reaction mixture is then heated at 150 to 190°C for 1 to 25 hours while water is distilled off.

2. A process as claimed in claim 1, wherein the molar ratio of diisopropanolamine to sulfuric acid is maintained at from 1:1.0 to 1:3.0.

3. A process as claimed in claim 1, wherein the water-containing 2,6-dimethylmorpholine is dried with concentrated sodium hydroxide solution at below 40°C.

4. A process as claimed in claim 3, wherein the dilute sodium hydroxide solution obtained as a result of drying the 2,6-dimethylmorpholine with concentrated sodium hydroxide solution is used for working up the sulfuric acid-containing reaction mixture of a subsequent batch.

## Revendications

1. Procédé de préparation de 2,6-diméthylmorpholine à teneur élevée en isomères cis, par cyclisation de diisopropanolamine avec de l'acide sulfurique, traitement du produit de réaction avec de la lessive de soude, distillation et séchage du produit brut obtenu, caractérisé par le fait que la diisopropanolamine, d'une teneur en eau de 0 à 20%, et 90 à 120% d'acide sulfurique en excès sont dosés simultanément, avec agitation, sans mesures de refroidissement, dans un milieu de réaction, de manière que la température dans le milieu de réaction s'élève jusqu'à 85 à 170°C et qu'on chauffe ensuite, pendant 1 à 25 h, à 150 à 190°C, avec élimination de l'eau par distillation.

2. Procédé selon la revendication 1, caractérisé par le fait qu'on maintient un rapport molaire diisopropanolamine/acide sulfurique de 1/1,0 à 1/3,0.

3. Procédé selon la revendication 1, caractérisé par le fait qu'on sèche, en dessous de 40°C, la 2,6-diméthylmorpholine avec une lessive de soude concentrée.

4. Procédé selon la revendication 3, caractérisé par le fait qu'on utilise la lessive de soude diluée, obtenue par séchage de la 2,6-diméthylmorpholine avec de la lessive de soude concentrée, lors des phases suivantes de réaction pour le traitement du produit de réaction sulfaté.